## Europäisches Patentamt

## European Patent Office

## Office européen des brevets

(11) Publication number: **0 186 363**

**B1**

(12) **EUROPEAN PATENT SPECIFICATION**

(45) Date of publication of patent specification: **14.11.90**

(51) Int. Cl.[5]: **C 07 F 15/00, C 09 B 57/10, A 61 K 31/28**

(21) Application number: **85308933.2**

(22) Date of filing: **09.12.85**

---

(54) **Platinum complexes.**

---

(30) Priority: **10.12.84 US 680044**

(43) Date of publication of application:
**02.07.86 Bulletin 86/27**

(45) Publication of the grant of the patent:
**14.11.90 Bulletin 90/46**

(84) Designated Contracting States:
**AT BE CH DE FR GB IT LI LU NL SE**

(56) References cited:
**US-A-4 490 543**

**CHEMICAL ABSTRACTS, vol. 103, no. 18, 4th November 1985, page 619, abstract no. 151020c, Columbus, Ohio, US**
**Ulmanns Encyclopädie der technischen Chemie, 4. Auflage, Band 16, 1978, Seiten 553-567**
**Christen H.R., Grundlagen der organischen Chemie, 1970, Seite 863**

**The file contains technical information submitted after the application was filed and not included in this specification**

(73) Proprietor: **JOHNSON MATTHEY, INC.,**
**4 Malin Road**
**Malvern Pennsylvania 19355 (US)**

(72) Inventor: **Teicher, Beverly A.**
**135 Hunting Road**
**Needham, MA 02192 (US)**
Inventor: **Richmond, Robert C.**
**Dept. Radiation Therapy Mary Hitchcock Hospital**
**Hanover, HN 03755 (US)**
Inventor: **Chen, Lan Bo**
**184 E. Emerson Road**
**Lexington, MA 02173 (US)**

(74) Representative: **Arthur, Bryan Edward et al**
**Withers & Rogers 4 Dyer's Buildings Holborn London EC1N 2JT (GB)**

---

Courier Press, Leamington Spa, England.

**Description**

The present invention is concerned with a new group of Pt complexes which demonstrate antitumor activity. The complexes also possess radiosensitizing activity whereby cells or tissues can be made more sensitive to killing effects of ionizing radiation when the complexes of the invention are used before, during, or after irradiation.

Among the known antitumor and radiosensitizing compounds are cisplatin (cis-diamine dichloroplatinum II, known for convenience as CDDP) and bis(5-aminofluorescein) dichloroplatinum II described in US patent 4,490,543.

The present invention comprises a (+)-charged dye transition metal complex for use as radiosensitizer which is the reaction product of a tetrachloroplatinate or cis-bis(acetonitrile) dichloroplatinum with a rhodamine-type dye molecule or a carbocyanine-type dye molecule. Where rhodamine 123 is used to prepare the complex the product is believed to be of formula Pt(Rh-123)$_2$.

The complexes of the invention may be made by reacting the above-mentioned platinum compound with rhodamine 123 or an other (+)-charged rhodamine dye or a cyanine dye such as: 3,3'-diethylthia-dicarbocyanine iodide, 3,3'-diethyloxatricarbocyanine iodide (known as 027), 3,3'-diethyloxa-dicarbocyanine iodide, 3,3'-diethylthitricarbocyanine iodide, the dye known as stains all (SA), or decaqualinium chloride (known as Deca).

Typically suitable platinum compounds include potassium tetrachloroplatinate (II) or like alkali metal tetrahaloplatinate (II); or *cis-bis*(acetonitrile)dichloroplatinum (II)

The reaction between the platinum compound and the rhodamine 123, or equivalents thereof, may be represented as follows, using potassium tetrachloroplatinate and rhodamine 123 for purposes of illustration:

$$KPtCl_4 + 2\ Rh\text{-}123 \rightarrow PtCl_2(Rh\text{-}123)_2 + 2KCl$$

The reaction is conveniently carried out by adding an organic or aqueous solution of the rhodamine 123 or equivalent to an organic or aqueous solution of the platinum compound, preferably while stirring, and allowing the reaction mixture to stand at room temperature (20—25°C) until the desired complex precipitates out. The precipitate may be separated and washed in conventional fashion to provide the product in an essentially pure state. Heat may be applied in some instances to facilitate the desired complexing reaction but usually it is adequate to employ temperatures in the order of 20—25°C. The reaction is usually completed in 10—24 hours.

In some cases the molar ratio of dye to platinum compound is 2:1; in other cases equimolar amounts of the reactants may be used although this is not critical. In any case, not more than 25% molar excess of either reactant is usually employed.

The present complexes demonstrate antitumor activity as shown on standard tests using MB 49 bladder carcinoma, P388 and L1210 leukemias and Lewis lung carcinoma. Based on the degree of activity demonstrated in the experimental tumors, the present complexes should be useful for their antitumor activity in essentially the same way, including dosages and mode of administration, as known antitumor Pt-complexes, e.g. cisplatin.

The invention is illustrated, but not limited, by the following:

Example 1

Potassium tetrachloroplatinate (II) (415 mg, 0.001 mole) was dissolved with stirring in 20 ml of distilled, deionized water. To this solution was added slowly with stirring a solution of rhodamine-123 (76 mg, 0.0022 mole) in 15 ml of distilled, dionized water. The reaction vessel was covered to protect the reaction mixture from light. The reaction was allowed to stir at room temperature (20°C) overnight, then was allowed to stand for 4 hours at 4°C. The dark red precipitate was collected by suction filtration and washed with 40 ml portions of ice cold 1 N hydrochloric acid, ice cold distilled ionized water, ice cold methanol and ice cold diethyl ether. The yield ranged from 45—55°. The composition of the product (Pt(Rh-123)) was confirmed by elemental analysis as shown in Table 1.

Example 2

*Cis-bis* (acetonitrile)dichloroplatinum (II) (392 mg, 0.001 mole) was dissolved with stirring in 20 ml of chloroform. To this solution was added slowly with stirring a solution of rhodamine-123 (762 mg, 0.0022 mole) in 15 ml of chloroform. The reaction vessel was covered to protect the reaction mixture from light. The reaction mixture was allowed to stir overnight at room temperature, then as concentrated solution was allowed to stand for 4 hours at 4°C, then the dark red precipitate was collected by suction filtration and washed with 40 ml portions of ice cold 1 N hydrochloric acid, ice cold distilled dionized water, ice cold methanol and ice cold diethyl ether. The yield ranged from 80—90%. The composition of the product (Pt(Rh-123)$_2$ was confirmed by elemental analysis as set forth in Table 1.

TABLE 1
Elemental Analysis of Pt(Rh-123)$_2$

|  | %C | %H | %N | %O | %Cl | %Pt |
|---|---|---|---|---|---|---|
| calculated for 5H$_2$O | 45.21 | 3.79 | 5.02 | 8.60 | 12.71 | 17.49 |
| found: |  |  |  |  |  |  |
| Example 1 | 45.13 | 3.96 | 5.01 | — | — | 17.45 |
| Example 2 | 47.42 | 3.60 | 5.27 | — | — | 18.34 |

Other complexes according to the invention were prepared generally as described in Examples 1 or 2 except that 3,3'-diethyloxatri-carbocyanine iodide (027), stains all (SA) and decaqualinium chloride (Deca) were used as the (+)-charged dye components in lieu of the rhodamine-123 to give products which may be identified for convenience as Pt 027, Pt SA and Pt Deca, respectively. The antitumor activity of Pt(Rh-123)$_2$ and these other complexes was compared with that of a known platinum complex and the dye components themselves as shown hereinafter.

Example 3

Antitumor activity for Pt(Rh-123)$_2$ was assessed in four transplantable tumors: (1) MB49 bladder carcinoma, (2) P388 leukemia, (3) L1210 leukemia, and (4) Lewis lung carcinoma. In all cases, 10$^6$ tumor cells were implanted intraperitoneally in mice on day 0 and drug treatment was begun on day 1.

The mice were thereafter treated with a non-toxic dose of Pt(Rh-123) or other (+)-dye Pt complex referred to above on an every other day schedule for days 1, 3, 5, 7, 9 and 11. The animals did not lose weight over the treatment period.

Pt(Rh-123)$_2$ and the other (+)-dye Pt complexes on this schedule demonstrated significant activity in all of the test tumors as shown in Table 2. The antitumor activity of these complexes was comparable to or better than that for the known complex, cis-diamminedichloro-platinum (II) (CDDP). Activity data is also provided in Table 2 for the dye components themselves to show the significant improvement which results from complexing according to the invention.

TABLE 2
Survival of mice bearing various tumors treated with various Pt complexes

| Drug | Dos, qd 2×6 | Tumor %T/C | | | |
|---|---|---|---|---|---|
|  | (mg/kg) | P388 | L1210 | MB49 | Lewis Lung |
| CDDP | 4.5 | 125 | 175 | 147 | 200 |
| Pt(Rh-123)$_2$ | 75 | 155 | 225 | 185 | 82 |
| Pt027 | 20 | 191 | 238 | 155 | 127 |
| PtSA | 3 | 155 | 225 | 179 | 136 |
| PtDeca | 10 | 127 | 250 | 197 | 179 |
| Rh-123 | 25 | 25 | 13 | 125 | 96 |
| 027 | 1.8 | 15 | 0 | 130 | 74 |
| SA | 1.0 | 22 | 25 | 155 | 110 |
| Deca | 4 | 36 | 38 | 1 | 54 |

P388 and L1210 are mouse leukemias. MB49 is a mouse bladder carcinoma and Lewis lung is a mouse lung carcinoma.

Example 4

*Radiosensitization Activity: In Vivo.* The Lewis lung tumor was grown in B6D2F1/J male mice (Jackson Laboratory) 8 to 10 weeks of age. There were seven animals in each group and the experiment was done twice. For the experiments, $2 \times 10^6$ tumor cells prepared from a brie of several stock tumors were implanted subcutaneously in the flanks of mice. When the tumors were approximately 50mm$^3$ in volume (about 1 week after tumor cell implantation, the animals were treated with a single dose of Pt(Rh-123) of 100 mg/kg or 200 mg/kg administered by i.p. injection. The animals showed no toxicity from this dose of Pt(Rh-123)$_2$.

One hour later the tumor-bearing limb was given a single dose of x-rays of 1000, 2000 or 3000 rads with a Gamma Cell 40 (Atomic Energy of Canada, dose rate; 88 rad per minute). The shielded portion of the animal received less than 2 percent of the delivered dose. Animals were anesthesized during the radiation treatment.

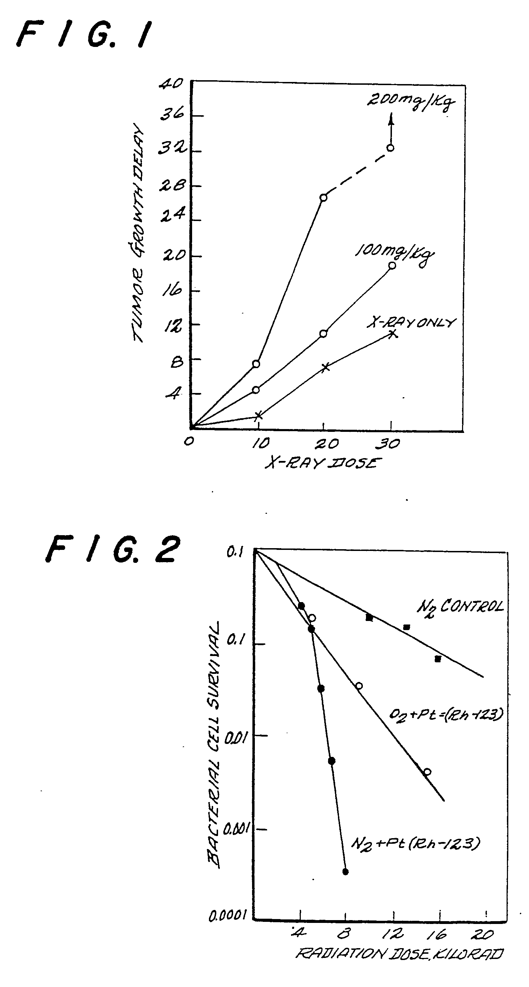

To determine the delay in tumor growth, the progress of each tumor was measured thrice weekly until it reached a volume of 500 mm$^3$. Untreated Lewis lung tumors reached 500 mm$^3$ in about 14 days. The tumor growth delay results are shown in Figure 1. The dose modifying factor obtained with 100 mg/kg of Pt(Rh-123)$_2$ was 1.6 and the dose modifying factor obtained with 200 mg/kg of Pt(Rh-123) was 2.1. To determine the dose modifying factor, zero growth delay was assumed with 0 rad and that factor which related the control dose of the same growth delay achieved with 1000 rads was calculated.

## Example 5

*Radiosensitization Activity. In Vitro After Heating.* A 200 4 µM solution of Pt(Rh-123) was heated to 90°C in phosphate buffered normal saline. The resulting preparation was superior radiosensitizer of hypoxic bacteral cells. Heating produced a preparation which was stable for many days (see Figure 1).

As shown in Figure 2, the preheated drug preparation was not a radiosensitizer of oxygenated cells but produced an enhancement of greater than 3-fold in hypoxic cells.

## Claims

1. A (+)-charged dye transition metal complex for use as a radiosensitizer which is the reaction product of a tetrachloroplatinate or cis-bis(acetonitrile) dichloroplatinum with a rhodamine-type dye molecule or a carbocyanine-type dye molecule.

2. A complex according to claim 1 wherein the dye molecule is rhodamine-123.

3. A method of preparing a complex according to claim 1 or 2 which comprises reacting a tetrachloroplatinate or cis-bis(acetonitrile) dichloroplatinum with the dye.

## Patentansprüche

1. Ein (+)-geladener Farbstoff/Übergangsmetall-Komplex zur Verwendung als ein Radiosensibilsator, der das Produkt ist aus einer Umsetzung von Tetrachlorplatinat oder cis-Bis-(acetonitril)-dichlorplatin mit einem Farbstoffmolekül vom Rhodamin-Typ oder einem Farbstoffmolekül vom Carbocyanin-Typ.

2. Komplex nach Anspruch 1, bei dem das Farbstoffmolekül Rhodamin-123 ist.

3. Verfahren zur Herstellung eines Komplexes nach Anspruch 1 oder 2, welches das Umsetzen von Tetrachlorplatinat oder cis-Bis-(acetonitril)-dichlorplatin mit dem Farbstoff umfaßt.

## Revendications

1. Complexe colorant chargé positivement-métal de transition, destiné à être utilisé comme radiosensibilisant, qui est le produit de réaction d'un tétrachloroplatinate ou du cis-bis-(acétonitrile)-dichloroplatine avec une molécule de colorant du type rhodamine ou une molécule de colorant du type carbocyanine.

2. Complexe suivant la revendication 1, dans lequel la molécule de colorant est la rhodamine-123.

3. Procédé de préparation d'un complexe suivant la revendication 1 ou 2, qui consiste à faire réagir un tétrachloroplatinate ou le cis-bis-(acétonitrile)-dichloroplatine avec le colorant.

# F I G. 1

# F I G. 2